**Europäisches Patentamt**

(19) **European Patent Office**

**Office européen des brevets**

(11) Veröffentlichungsnummer: **0 259 399 B1**

(12) **EUROPÄISCHE PATENTSCHRIFT**

(45) Veröffentlichungstag der Patentschrift:
17.04.91 Patentblatt 91/16

(51) Int. Cl.⁵: **A61F 9/00, A61B 3/10,** G02B 27/14

(21) Anmeldenummer: 87901391.0

(22) Anmeldetag: 12.03.87

(86) Internationale Anmeldenummer:
PCT/DE87/00110

(87) Internationale Veröffentlichungsnummer:
WO 87/05495 24.09.87 Gazette 87/21

(54) **EINRICHTUNG ZUM EINSPIEGELN EINES LASERSTRAHLS IN EIN SPALTLAMPENGERÄT.**

(30) Priorität: 12.03.86 DE 3608287

(43) Veröffentlichungstag der Anmeldung:
16.03.88 Patentblatt 88/11

(45) Bekanntmachung des Hinweises auf die
Patenterteilung:
17.04.91 Patentblatt 91/16

(84) Benannte Vertragsstaaten:
CH DE FR GB IT LI

(56) Entgegenhaltungen:
WO-A-85/00966
WO-A-85/01870
FR-A- 2 464 704
Patent Abstracts of Japan, volume 5, No 192
(E-85) (864), 08 December 1981, see figure and
abstract, & JP, A 56114390 (SUMITOMO DENKI
KOGYO K.K) 08 Sebtember 1981

(73) Patentinhaber: G. RODENSTOCK
INSTRUMENTE GMBH
Drachenseestr. 10 - 12
W-8000 München 70 (DE)

(72) Erfinder: REIS, Werner
Dachauerstr. 407
W-8000 München 50 (DE)
Erfinder: TRILLER, Adolf
Regerstr. 23
W-8032 Lochham (DE)

(74) Vertreter: Münich, Wilhelm, Dr. et al
Kanzlei Münich, Steinmann, Schiller
Willibaldstrasse 36/38
W-8000 München 21 (DE)

# Beschreibung

## Technisches Gebiet

Die Erfindung bezieht sich auf eine Einrichtung zum Einspiegeln eines Laserstrahls in ein Spaltlampengerät gemäß dem Oberbegriff des Patentanspruchs 1.

## Stand der Technik

Eine Einrichtung zum Einspiegeln eines Laserstrahls in ein Spaltlampengerät ist beispielsweise aus dem Deutschen Gebrauchsmuster 72 25 429 bekannt. Bei dieser Einrichtung wird der Laserstrahl in das Gehäuse der Spaltleuchte eingeleitet und durch die Spaltleuchte auf das zu untersuchende bzw. zu behandelnde Auge geführt. Diese Einrichtung erfordert damit einen Eingriff in die Spaltleuchte, so daß beispielsweise ein Laser an einem bestehenden Spaltlampengerät nicht ohne weiteres nachzurüsten ist. Darüberhinaus ist es optisch aufwendig, sowohl den Laserstrahl als auch das Licht der Spaltleuchte, deren Wellenlängenbereich stark unterschiedlich sein kann, durch die gleichen Bauelemente zu führen. Ferner ist es praktisch unmöglich, im Strahlengang der Spaltleuchte Elemente anzuordnen, die den Laserstrahl selektiv beeinflußen, beispielsweise aufweiten.

Es ist deshalb in dem Deutschen Gebrauchsmuster 79 25 899 bzw. der prioritätsgleichen FR-A-2 464 704, von der bei der Formulierung des Oberbegriffs des Anspruchs 1 ausgegangen wird, vorgeschlagen worden, in Strahlrichtung nach dem Umlenkprisma der Spaltleuchte ein weiteres, kleines Prisma anzuordnen, das den Laserstrahl auf das zu untersuchende bzw. zu behandelnde Auge umlenkt. Bei dieser Einrichtung ist zwar kein Eingriff in die Spaltleuchte erforderlich, nachteilig ist aber, daß der Laserstrahl und das Licht der Spaltleuchte nicht gleichachsig geführt sind. Deshalb wirken sich Schwenk und Drehbewegungen, die beispielsweise mittels eines sogenannten Mikromanipulators durchgeführt werden, beim Prisma, das den Laserstrahl umlenkt, anders als bei dem Prisma aus, mit dem das Licht der Spaltleuchte auf das Auge umgelenkt wird.

Weiterhin ist vorgeschlagen worden, den Laserstrahl unabhängig von der Spaltleuchte beispielsweise durch das Spaltlampenmikroskop (internationale Anmeldung DE 84/00179) oder durch ein gesondertes, unabhängig bewegbares Einkoppelprisma auf das Auge zu lenken. Bei diesen Anordnungen ist es jedoch nachteilig, daß bei Manipulationen an der Spaltleuchte der durch die Spaltleuchte beleuchtete Bereich nicht mehr vollständig mit dem Zielpunkt des Lasers zusammenfallen muß.

## Darstellung der Erfindung

Der Erfindung liegt die Aufgabe zu Grunde, eine Einrichtung zum Einspiegeln eines Laserstrahls in ein Spaltlampengerät anzugeben, bei der bei Manipulation des Laserflecks kein seitliches Auswandern des Laserstrahls gegenüber der Spaltbeleuchtung auftritt und bei der keine Eingriffe in die eigentliche Spaltleuchte erforderlich sind.

Eine erfindungsgemäße Lösung.dieser Aufgabe ist mit ihren Weiterbildungen in den Patentansprüchen gekennzeichnet.

Überraschenderweise gelingt eine Lösung der erfindungsgemäß gestellten Aufgabe dadurch, daß von einer Einrichtung zum Einspiegeln eines Laserstrahls gemäß dem Oberbegriff des Patentanspruchs 1 ausgegangen, und diese Einrichtung dadurch weitergebildet wird, daß sich die beiden Spiegelflächen des Umlenkelements durchsetzen. Vor dem Umlenkelement verläuft demnach der Strahlengang des Laserstrahls in der Verlängerung der optischen Achse der Spaltleuchte. Da die optische Achse der Spaltleuchte bzw. deren Verlängerung für beide Strahlengänge gemeinsam benutzt wird, tritt bei Manipulation des Laserflecks kein Auswandern des Laserflecks gegenüber der Spaltbeleuchtung aus. Es ist deshalb möglich, Mikromanipulatoren, wie sie bei bekannten Spaltlampengeräten des Anmelders verwendet werden, ohne Änderung zu verwenden.

Der erfindungsgemäße Grundgedanke kann selbstverständlich fü Spaltlampengeräte verwendet werden, bei denen der Patient sitzt und die optische Achse der Spaltleuchte vertikal verläuft. In gleicher Weise ist es möglich, den erfindungsgemäßen Grundgedanken auch bei Spaltlampengeräten zu verwenden, bei denen der Patient liegt, und die optische Achse der Spaltleuchte zunächst horizontal verläuft.

Das Umlenkelement, das die beiden aufeinander zugerichteten Strahlengänge der Spaltleuchte und des Lasers auf das zu untersuchende bzw. zu behandelnde Auge umlenkt, kann beispielsweise aus zwei einfachen Spiegeln bestehen. Besonders vorteilhaft ist jedoch die im Anspruch 2 gekennzeichnete Gestaltung des Umlenkelements, gemäß der das Umlenkelement ein aus vier gleichen Prismen bestehender Teilerwürfel ist. Die Grenzflächen der Prismen sind mindestens teilweise verspiegelt, so daß die von gegenüberliegenden Seiten des Teilerwürfels eingeleiteten Strahlen der Spaltleuchte und des Laserstrahls um 90° auf das Auge umgelenkt werden.

Zur Verminderung von Reflexionsverlusten ist es vorteilhaft, wenn die vier Prismen, die den Teilerwürfel blden, gemäß Anspruch 3 miteinander verkittet sind.

Die Verspiegelung der einzelnen Prismen kann gemäß Anspruch 4 dabei derart sein, daß der Laserstrahl innerhalb des Strahlengangs der Spaltleuchte verläuft.

Ferner ist es auch möglich, die Prismen oder Spiegel wellenlängenselektiv zu beschichten, wenn sich die Wellenlänge des Lasers wesentlich von der Wellenlänge des Lichts der Spaltleuchte unterscheidet (Anspruch 5).

In jedem Falle ist es jedoch möglich, den Querschnitt des Laserstrahls beliebig zu beeinflussen. Beispielsweise können vor dem Umlenkelement strahlablenkende Elemente vorgesehen sein, die so angesteuert werden, daß der Laserstrahl ein bestimmtes Muster, beispielsweise einen großen Koagulationsfleck oder einen auf der Cornea durchzuführenden Schnitt "austastet".

Im Anspruch 6 ist gekennzeichnet, daß der Strahlengang des Lasers zunächst versetzt zur optischen Achse der Spaltleuchte geführt ist, und erst ein kurz vor dem Umlenkelement angeordnetes Versetzprisma den Strahlengang des Lasers in die Verlängerung der optischen Achse der Spaltleuchte umlenkt. Diese Anordnung hat den Vorteil, daß der zwischen optischer Achse des Strahlengangs des Laserstrahls und Spaltleuchtenmikroskop zur Verfügung stehende Platz vergrößert wird, so daß beispielsweise ein Argonlaser direkt auf die Spaltleuchte aufgesetzt werden kann.

Im Anspruch 7 ist eine vorteilhafte Ausgestaltung des Strahlengangs nach dem Umlenkelement gekennzeichnet.

In jedem Falle hat die erfindungsgemäße Einrichtung jedoch den Vorteil, daß durch die Einspiegelung des Laserstrahls und der Spaltleuchten-Beleuchtung in der gleichen Achse jedoch von gegenüberliegenden Seiten kein relativer Auslenkversatz zwischen Laserfleck und Beleuchtungsstrahlengang auftritt. Die erfindungsgemäße Einrichtung erlaubt damit die Verwendung bekannter Mikromanipulatoren zur Beeinflussung des Auftreffpunkts des Laserstrahls.

Darüberhinaus können Maßnahmen zur Beeinflussung des Strahlquerschnitts, wie Einsatz einer Variooptik eines Taumel-Elements zur Austastung eines größeren Gebiets (Patentanmeldung P 35 32 464.3) ohne weiteres weiterverwendet werden.

Die erfindungsgemäße Einrichtung eignet sich darüberhinaus auch für Geräte, bei denen ein Ziel- und Behandlungsstrahl vorgesehen ist, beispielsweise für Neodym-YAG-Laser mit einem Helium-Neon-Zielstrahllaser, für Laser mit einer Wellenlänge von 3 m für Excimer-Laser.

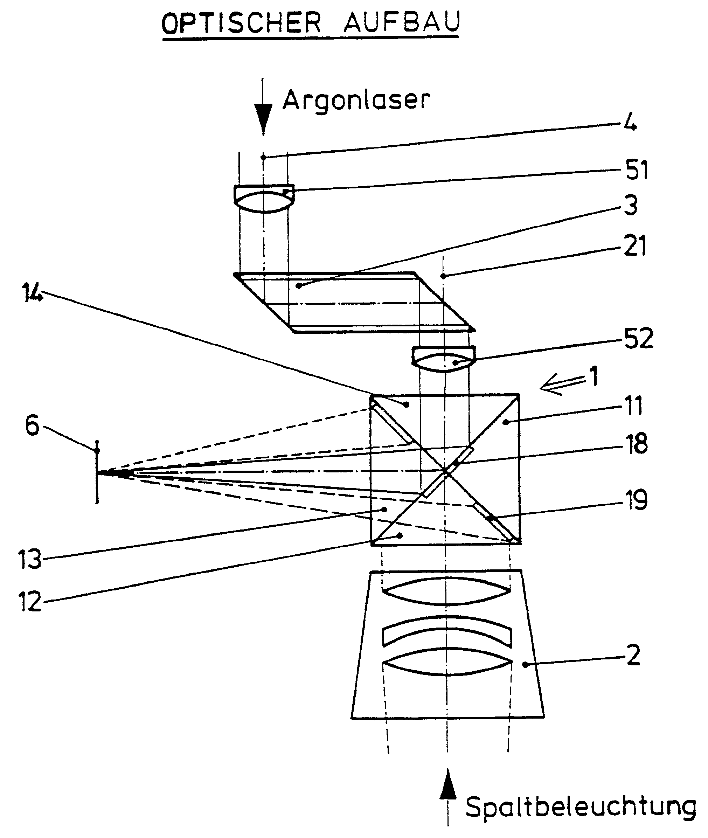

## Kurze Beschreibung der Zeichnung

Die Erfindung wird nachstehend anhand eines Ausführungsbeispiels unter Bezugnahme auf die Zeichnung näher beschrieben, deren einzige Figur einen Querschnitt durch eine erfindungsgemäße Einrichtung zeigt.

## Weg zur Ausführung der Erfindung

Die erfindungsgemäße Einrichtung weist ein Umlenkelement 1 auf, das auf eine Spaltleuchte 2 aufgesetzt ist. Bei dem gezeigten Ausführungsbeispiel ist das Umlenkelement 1 ein aus vier gleichen Prismen 11, 12, 13 und 14 bestehender Teilerwürfel. Auf der der Spaltleuchte gegenüberliegenden Seite ist ein weiteres Prisma 3 vorgesehen, daß den Strahlengang 4 eines nicht dargestellten Lasers, beispielsweise eines Argon-Lasers so umlenkt, daß er in der Verlängerung der optischen Achse 21 der Spaltleuchte 2, aber von der gegenüberliegenden Seite des Teilerwürfels 1 in diesen eintritt. Im Strahlengang 4 des Lasers sind ferner strahlbeeinflussende Elemente 51 und 52 vorgesehen, wobei das als Achromat ausgebildete Element 52 an die Austrittsfläche des zweiten Prismas 3 angekittet. Das Element 52 fokussiert den Laserstrahl auf das zu untersuchende bzw. zu behandelnde Auge 6.

Die Grenzflächen der Prismen 11 bis 14, die den Teilerwürfel 1 bilden, sind mit Spiegelschichten 18 und 19 bedampft, wobei die "innenliegende" Spiegelfläche 18 das Laserlicht und die ringförmige Spiegelschicht 19 das Licht der Spaltleuchte auf das Auge 6 derart umlenkt, daß das Laserlicht "innerhalb" des Spaltleuchtenlichts verläuft.

Die in der Figur dargestellte Einrichtung hat eine Reihe von Vorteilen :

Durch die gemeinsame optische Achse 21 des Laserlichts und des Spaltleuchtenlichts bewirken Manipulationen des Laserflecks kein seitliches Auswandern des Laserstrahls gegenüber der Spaltbeleuchtung. Damit ist es möglich, den Teilerwürfel 1 und das Prisma 3 mit aufgekittetem Fokussierachromat gemeinsam mittels eines bekannten Mikromanipulators zu bewegen.

Darüberhinaus tritt durch den für das Laserlicht und das Spaltleuchtenlicht gemeinsamen Teilerwürfel, der in der Verlängerung der optischen Achse der Spaltleuchte angeordnet ist, keine Vignettierung für den Beobachter auf, der beispielsweise einen Operationsvorgang durch das Spaltlampenmikroskop beobachtet.

Durch das Umlenkprisma 3, das die Achse des Laserstrahls gegenüber der Achse der Spaltleuchte versetzt, wird der in Richtung auf die Spaltleuchte zur Verfügung stehende Platz erhöht, so daß beispielsweise ein Argonlaser direkt aufgesetzt werden kann.

Vorstehend ist die Erfindung anhand eines Ausführungsbeispiels näher beschrieben worden. Innerhalb des allgemeinen Erfindungsgedankens sind selbstverständlich die verschiedensten Modifikationen möglich :

So ist es möglich, den Teilerwürfel durch ein anderes optisches Element zu ersetzen, daß das Licht der Spaltleuchte sowie das Licht des Laserstrahls, das von der gegenüberliegenden Seite eintritt, auf das Auge umlenkt. Ferner können die Spiegelschichten 18 und 19 auf den den Teilerwürfel 1 bildenden Prismen 11 bis 14 auch durch wellenlängenselektive Schichten ersetzt werden.

Natürlich ist es auch möglich, an Stelle des im Ausführungsbeispiel verwendeten Argonlasers einen anderen Laser, beispielsweise einen Excimer-Laser, eine Neodym-YAG-Laser oder einen Laser mit einer Wellenlänge von 3 m zu verwenden. Bei diesen Lasern kann ohne Schwierigkeiten auch zusätzlich ein Zielstrahllaser eingesetzt werden.

In jedem Falle hat die erfindungsgemäße Einrichtung den Vorteil, daß anders als bei den bekannten Einrichtungen, bei denen das Licht durch die Spaltleuchte geführt wird, vor dem Umlenken des Laserstrahls beliebige strahlbeeinflussende Elemente vorgesehen werden können, die beispielsweise die Fleckgröße durch Austasten oder mittels einer Variooptik verändern. Dabei kann insbesondere auf eine "Austasteinrichtung" zurückgegriffen werden, wie sie in der Deutschen Patentanmeldung 35 32 464.3 beschrieben ist.

## Ansprüche

1. Einrichtung zum Einspiegeln eines Laserstrahls in den Strahlengang einer Spaltleuchte (2) eines Spaltlampengeräts, mit einem Umlenkelement (1), das zwei einen 90°-Winkel einschließende Spiegelflächen (18, 19) aufweist, von denen die eine das Licht der Spaltleuchte und die andere den Laserstrahl (4) koaxial mit dem Spaltlampen-Licht auf das zu untersuchende bzw. zu behandelnde (6) Auge umlenkt, dadurch **gekennzeichnet**, daß sich die beiden Spiegelflächen (18, 19) des Umlenkelements (1) durchsetzen, und daß der Laserstrahl (4) in der Verlängerung der optischen Achse (21) der Spaltleuchte (2) auf die entsprechende Spiegelfläche (18) geführt ist.

2. Einrichtung nach Anspruch 1, dadurch **gekennzeichnet**, daß das Umlenkelement ein Teilerwürfel ist, der aus vier gleichen Prismen (11...14) besteht, deren Grenzflächen mindestens teilweise reflektierend beschichtet sind.

3. Einrichtung nach Anspruch 1, dadurch **gekennzeichnet**, daß die vier Prismen miteinander verkittet sind.

4. Einrichtung nach Anspruch 1 oder 3, dadurch **gekennzeichnet**, daß die einzelnen Prismen derart verspiegelt sind, daß der Laserstrahl innerhalb des Strahlengangs der Spaltleuchte verläuft.

5. Einrichtung nach einem Ansprüche 1 bis 3, dadurch **gekennzeichnet**, daß die einzelnen Prismen wellenlängenselektiv beschichtet sind.

6. Einrichtung nach einem der Ansprüche 1 bis 5, dadurch **gekennzeichnet**, daß der Strahlengang (4) des Lasers zunächst versetzt zur optischen Achse (21) der Spaltleuchte (2) geführt ist, und zwei Spiegel (3) den Strahlengang des Lasers in die Verlängerung der optischen Achse der Spaltleuchte umlenkt.

7. Einrichtung nach Anspruch 6 dadurch **gekennzeichnet**, daß ein Versetzprisma (3) den Strahlengang (4) des Lasers umlenkt.

8. Einrichtung nach Anspruch 6 oder 7, dadurch **gekennzeichnet**, daß nach dem Versetzprisma (3) ein optisches Fokussierelement (52) vorgesehen ist.

## Claims

1. A device to image a laser beam into the beam path of a slit lamp (2) of a slit lamp unit, with a reflecting element (1) which possesses two mirror surfaces (18, 19) enclosing an angle of 90° of which the one surface reflects the light of the slit lamp and the other the laser beam (4) coaxially with the slit lamp light onto the eye (6) to be examined or treated, characterized by the fact that the two mirror surfaces (18, 19) of the reflecting element (1) intermingle and that the laser beam (4) is led to the corresponding mirror surface (18) in the extension of the optical axis (21) of the slit lamp (2).

2. A device according to claim 1, characterized by the fact that the reflecting element is a beamsplitter comprising four equal prisms (11...14) whose boundary surfaces are at least partially reflection-coated.

3. A device according to claim 1, characterized by the fact that the four prisms are cemented to each other.

4. A device according to claims 1 or 3, characterized by the fact that the single prisms are silvercoated in such a way that the laser beam runs inside the beam path of the slit lamp.

5. A device according to any of the claims 1 to 3, characterized by the fact that the single prisms have a wavelength-selective coating.

6. A device according to any of the claims 1 to 5, characterized by the fact that the beam path (4) of the laser is first led displaced with respect to the optical axis (21) of the slit lamp (2) and that two mirrors (3) reflect the beam path of the laser into the extension of the optical axis of the slit lamp.

7. A device according to claim 6, characterized by the fact that a displacement prism (3) reflects the beam path (4) of the laser.

8. A device according to claims 6 or 7, characterized by the fact that an optical focusing element (52) is provided after the displacement prism (3).

## Revendications

1. Dispositif destiné à réfléchir un rayon laser dans le parcours des rayons d'une lampe à fente (2) d'un appareil à lampe à fente, comportant un élément déviateur (1) qui présente deux surfaces réfléchissantes (18, 19) formant un angle inclus de 90°, dont l'une dévie la lumière de la lampe à fente et l'autre le faisceau laser (4), coaxialement à la lumière de la lampe à fente, sur l'oeil (6) qu'il s'agit d'examiner ou de traiter, dispositif caractérisé en ce que les deux surfaces réfléchissantes (18, 19) de l'élément déviateur (1) s'intersectent et en ce que le faisceau laser (4) est guidé dans le prolongement de l'axe optique (21) de la lampe à fente (2) sur la surface réfléchissante (18) correspondante.

2. Dispositif selon la revendication 1, caractérisé en ce que l'élément déviateur est un bloc séparateur qui est constitué par quatre prismes (11 à 14) identiques, dont les surfaces de séparation sont ; au moins partiellement, revêtues d'une couche réfléchissante.

3. Dispositif selon la revendication 1, caractérisé en ce que les quatre prismes sont montés ensemble au moyen de mastic.

4. Dispositif selon la revendication 1 ou 3, caractérisé en ce que les prismes individuels sont traités de façon réfléchissante de manière telle que le faisceau laser s'étend à l'intérieur du parcours des rayons de la lampe à fente.

5. Dispositif selon une des revendications 1 à 3, caractérisé en ce que les prismes individuels sont revêtus de couche sélectives en fonction de la longueur d'onde.

6. Dispositif selon une des revendications 1 à 5, caractérisé en ce que le parcours (4) des rayons du laser est d'abord guidé avec un décalage par rapport à l'axe optique (21) de la lampe à fente (2) et en ce que deux miroirs (3) dévient le parcours des rayons du laser pour l'amener dans le prolongement de l'axe optique de la lampe à fente.

7. Dispositif selon la revendication 6, caractérisé en ce qu'un prisme (3) de décalage dévie le parcours (4) du faisceau du laser.

8. Dispositif selon la revendication 6 ou 7, caractérisé en ce que, en aval du prisme (3) de décalage est prévu un élément (52) optique de focalisation.

# OPTISCHER AUFBAU

Argonlaser

Spaltbeleuchtung